(19) Europäisches Patentamt
European Patent Office
Office européen des brevets

(11) Publication number : 0 624 589 A2

(12) **EUROPEAN PATENT APPLICATION**

(21) Application number : **94303321.7**

(22) Date of filing : **09.05.94**

(51) Int. Cl.[5] : **C07D 498/04**, C07D 498/12, A61K 31/42, A61K 31/35

(30) Priority : **11.05.93 US 60282**

(43) Date of publication of application : **17.11.94 Bulletin 94/46**

(84) Designated Contracting States : **AT BE CH DE DK ES FR GB GR IE IT LI LU MC NL PT SE**

(71) Applicant : **BRISTOL-MYERS SQUIBB COMPANY**
**P.O. Box 4000**
**Princeton, NJ 08543-4000 (US)**

(72) Inventor : **Fox, Rita T.**
**109 Farber Road,**
**Apt. 5B**
**Princeton, New Jersey (US)**
Inventor : **Godfrey, Jollie D., Jr.**
**16 Wickford Avenue**
**Trenton, New Jersey (US)**
Inventor : **Mueller, Richard H.**
**66 Lindbergh Road**
**Ringoes, New Jersey (US)**

(74) Representative : **Thomas, Roger Tamlyn et al**
**D. Young & Co.**
**21 New Fetter Lane**
**London EC4A 1DA (GB)**

(54) **Intermediates useful in the preparation of pyranyl cyanoguanidine derivatives and methods of preparing same.**

(57) Compounds of the formula

I

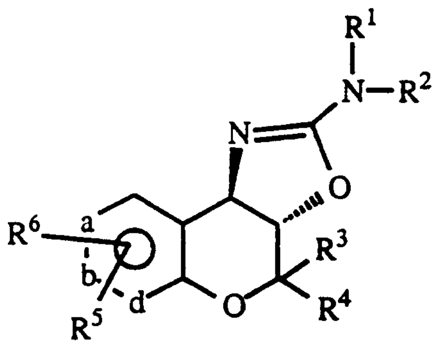

;

II

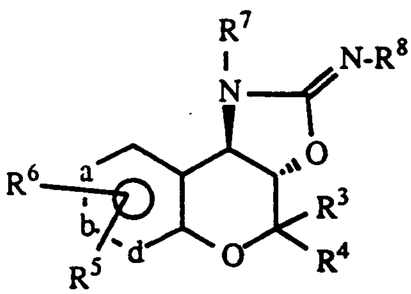

;

and

EP 0 624 589 A2

Jouve, 18, rue Saint-Denis, 75001 PARIS

III

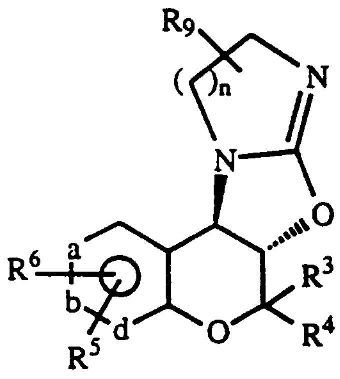

where a, b, d, and $R^1$ to $R^{10}$ are as defined herein. The compounds of formulae I, II and III are intermediates useful in the preparation of pyranyl cyanoguanidine derivatives.

The present invention relates to novel intermediates useful in preparing compounds having potassium channel activating activity, and processes for preparing the intermediates.

The present invention is directed to compounds of the formulae

I

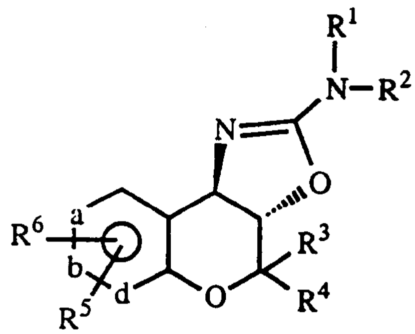

;

II

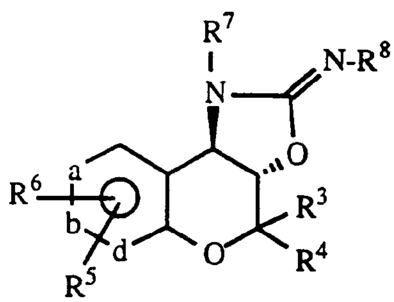

;

and

III

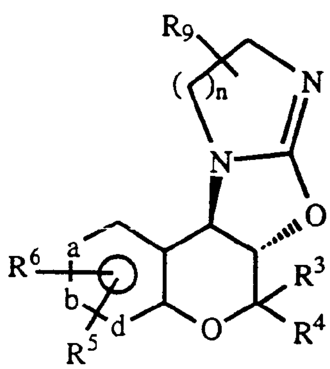

.

As used in formulae I, II, III and throughout the specification, the symbols have the following meanings:

a, b, and d are all carbon atoms or one of a, b and d is a nitrogen atom or -N(O)- and the others are carbon atoms;

$R^1$ and $R^2$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or $R^1$ and $R^2$ taken together with the nitrogen atom to which they are attached form 1-pyrrolidinyl, 1-piperidinyl, 1-azepinyl, 4-morpholinyl, 4-thiamorphilinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;

$R^3$ and $R^4$ are each independently hydrogen, alkyl or arylalkyl, or, $R^3$ and $R^4$ taken together with the

carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

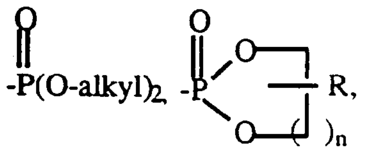

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^7$ and $R^8$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^9$ is hydrogen, alkyl, alkenyl, aryl, arylalkyl, cycloalkyl, (cycloalkyl)alkyl or an aryl group fused to 2 adjacent carbon atoms; and

n is an integer of 1 to 3.

The present invention provides novel compounds of formulae I, II and III and processes for preparing such compounds. Various processes are set out in the claims and described in detail below. Listed below are definitions of various terms used to describe the compounds of the instant invention. These definitions apply to the terms as they are used throughout the specification (unless they are otherwise limited in specific instances) either individually or as part of a larger group.

The term "alkyl" refers to straight and branched chain hydrocarbons, containing 1 to 8 carbons in the normal chain, preferably 1 to 5 carbons such as methyl, ethyl, propyl, butyl, pentyl, the various branched chain isomers thereof such as isopropyl, t-butyl, isobutyl, 4,4-dimethyl-pentyl, 2,2,4-trimethylpentyl, and the like as well as such groups including a halo-substituent, such as F, Br, Cl or I such as $CCl_3$ or $CF_3$, an alkoxy substituent, an aryl substituent, an alkylaryl substituent, a haloaryl substituent, a cycloalkyl substituent, a (cycloalkyl)alkyl substituent, a hydroxy substituent, an alkylamino substituent, an alkanoylamino substituent, an arylcarbonylamino substituent, a nitro substituent, a cyano substituent, a thiol substituent or an alkylthio substituent.

The terms "alkoxy" and "alkylthio" refer to such alkyl groups as described above linked to an oxygen atom or sulfur atom respectively.

The term "alkenyl" refers to such groups as described above for alkyl, further containing at least one carbon to carbon double bond.

The term "alkynyl" refers to such groups as described above for alkyl, further containing at least one carbon to carbon triple bond.

The term "cycloalkyl" as employed herein includes saturated cyclic hydrocarbon groups containing 3 to 7 ring carbons with cyclopropyl, cyclopentyl and cyclohexyl being preferred.

The term "halogen" or "halo" refers to chlorine, bromine, iodine and fluorine.

The term "aryl" refers to phenyl, 1-naphthyl, 2-naphthyl or mono substituted phenyl, 1-naphthyl, 2-naphthyl wherein said substituents is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, $-N(alkyl)_2$ wherein alkyl is of 1 to 4 carbons, $-CF_3$,

$-OCHF_2$, 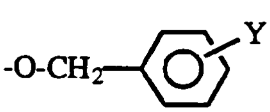 , 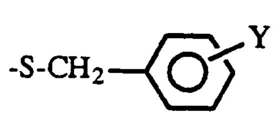

(wherein Y is hydrogen, alkyl of 1 to 4 carbons, alkoxy of 1 to 4 carbons, alkylthio of 1 to 4 carbons, halo, hydroxy or $-CF_3$), $-O-CH_2$-cycloalkyl, or $-S-CH_2$-cycloalkyl, and di-substituted phenyl, 1-naphthyl, 2-naphthyl wherein said substituents are selected from methyl, methoxy, methylthio, halo, $-CF_3$, nitro, amino, and $-OCHF_2$. Preferred aryl groups include unsubstituted phenyl and monosubstituted phenyl wherein the substituents are nitro, halo, $-CF_3$, alkyl, cyano or methoxy.

The term "heterocyclo" or "hetero" refers to fully saturated or unsaturated rings of 5 or 6 atoms containing

one or two oxygen and sulfur atoms and/or one to four nitrogen atoms provided that the total number of hetero atoms in the ring is 4 or less. The hetero ring is attached by way of an available carbon atom. Preferred monocyclic hetero groups include 2- and 3-thienyl, 2- and 3-furyl, 2-, 3- and 4-pyridyl, and imidazolyl. The term hetero also includes bicyclic rings wherein the five or six membered ring containing oxygen, sulfur and nitrogen atoms as defined above is fused to a benzene ring and the bicyclic ring is attached by way of an available carbon atom. Preferred bicyclic hetero groups include 4, 5, 6, or 7-indolyl, 4, 5, 6 or 7-isoindolyl, 5, 6, 7 or 8-quinolinyl, 5, 6, 7 or 8-isoquinolinyl, 4, 5, 6, or 7-benzothiazolyl, 4, 5, 6 or 7-benzoxazolyl, 4, 5, 6 or 7-benzimidazolyl, 4, 5, 6 or 7-benzoxadiazolyl and 4, 5, 6 or 7-benzofuranzanyl.

The term heterocyclo also includes such monocyclic and bicyclic rings wherein an available carbon atom is substituted with a lower alkyl of 1 to 4 carbons, lower alkylthio of 1 to 4 carbons, lower alkoxy of 1 to 4 carbons, halo, nitro, keto, cyano, hydroxy, amino, -NH-alkyl wherein alkyl is of 1 to 4 carbons, $-N(alkyl)_2$ wherein alkyl is of 1 to 4 carbons, $-CF_3$, or $-OCHF_2$ or such monocyclic and bicyclic rings wherein two or three available carbons have substituents selected from methyl, methoxy, methylthio, halo, $-CF_3$, nitro, hydroxy, amino and $-OCHF_2$.

The term "substituted amino" refers to a group of the formula $-NZ^1Z^2$ wherein $Z^1$ is hydrogen, alkyl, cycloalkyl, aryl, arylalkyl, (cycloalkyl)alkyl and $Z^2$ is alkyl, cycloalkyl, aryl, arylalkyl, (cycloalkyl)alkyl or $Z^1$ and $Z^2$ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, 1-pyrrolidinyl, 1-piperidinyl or 1-azepinyl substituted with alkyl, alkoxy, alkylthio, halo, trifluoromethyl or hydroxy.

The compounds of the present invention can have asymmetric centers at carbons 24 of benzopyran ring. Also, any one of the R's can have an asymmetric carbon. Consequently, compounds of formulae I, II and III can exist in diastereomeric forms or in mixtures thereof. The below described processes can utilize racemates, enantiomers or diastereomers as starting materials. When diastereomeric products are prepared, they can be separated by conventional chromatographic or fractional crystallization methods.

Compounds of formula I where $R^1$ is hydrogen and $R^2$ is other than hydrogen, may be prepared by reacting a compound of formula

IV

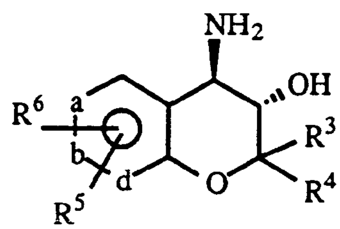

with a isocyanide dihalide of formula

V $R^2\text{-}N{=}C(X)_2$

(where $R^2$ is other than hydrogen and X is a halogen, preferably chlorine); in a solvent such as dichloromethane, 1,2 dichloroethane, acetonitrile, ethyl acetate or preferably an alcoholic solvent such as isopropyl alcohol or ethanol containing a tertiary amine such as diisopropylethylamine to form the compounds of formula I where $R^1$ is hydrogen and $R^2$ is other than hydrogen.

Alternatively, compounds of formula I where $R^1$ is hydrogen and $R^2$ is other than hydrogen, may be prepared by treatment of compounds of formula IV, with an isothiocyanate of the formula

VI $R^2\text{-}N{=}C{=}S$

where $R^2$ is other than hydrogen, such as 4-chlorophenylisothiocyanate to provide a thiourea of formula

VII

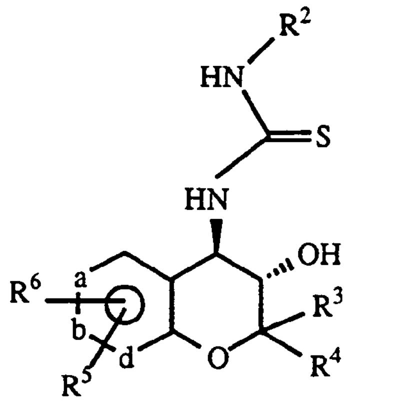

Subsequent treatment of the thiourea of formula VII with a carbodiimide such as 1-ethyl-3-(3-dimethylaminopropyl)-carbodiimide hydrochloride (WSC) provides the compounds of formula I where $R^1$ is hydrogen and $R^2$ is other than hydrogen.

To prepare compounds of formula I where $R^1$ and $R^2$ are other than hydrogen, a compound of formula IV is reacted with a compound of formula

VIII

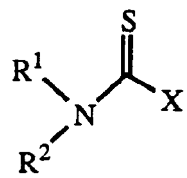

(where $R^1$ and $R^2$ are other than hydrogen and X is a halogen, preferably chlorine), to form a compound of formula

IX

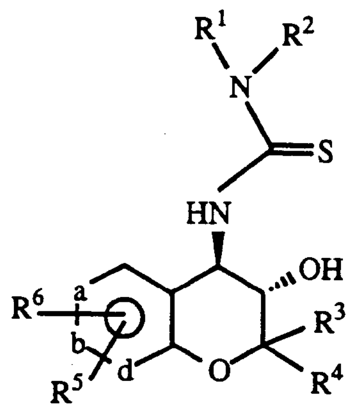

where $R^1$ and $R^2$ are other than hydrogen. Subsequent treatment of the thiourea of formula IX with a carbodiimide such as 1-ethyl-3-(3-dimethylaminopropyl)carbodiimide hydrochloride (WSC) provides the compounds of formula I where $R^1$ and $R^2$ are other than hydrogen.

To prepare compounds of formula I where $R^1$ and $R^2$ are hydrogen, a compound of formula IV is reacted with a compound of formula

X X-CN

where X is a halogen, preferably bromine or chlorine in an alcoholic solvent such as methanol.

Compounds of formula II where $R^8$ is other than hydrogen, may be prepared by reacting a compound of

formula

XI

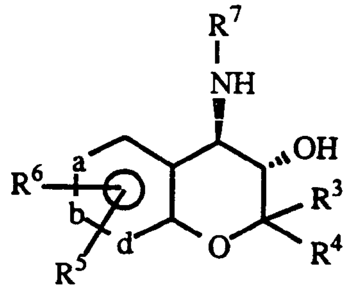

with a isocyanide dihalide of formula

XII $R^8$-N=C(X)$_2$

(where $R^8$ is other than hydrogen and X is a halogen, preferably chlorine); in a solvent such as dichloromethane, 1,2 dichloroethane, acetonitrile, ethyl acetate or preferably an alcoholic solvent such as isopropyl alcohol or ethanol containing a tertiary amine such as diisopropylethylamine to form the compounds of formula II, where $R^8$ is other than hydrogen.

Alternatively, compounds of formula II where $R^8$ is other than hydrogen may be prepared by treatment of compounds of formula XI with an isothiocyanate of the formula

XIII $R^8$-N=C=S

where $R^8$ is other than hydrogen, to provide a thiourea of formula

XIV

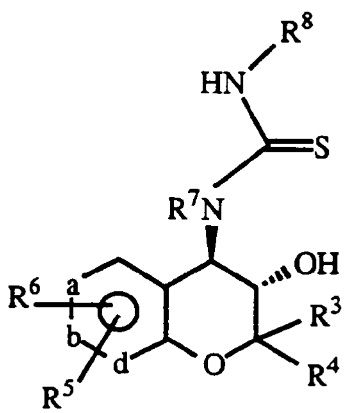

Subsequent treatment of the thiourea of formula XIV with a carbodiimide such as 1-ethyl-3-(3-dimethylaminopropyl)carbodiimide hydrochloride (WSC) provides the compounds of formula II, where $R^8$ is other than hydrogen.

To prepare compounds of formula II, where $R^8$ is hydrogen, a compound of formula X is reacted with a compound of formula XI.

The reactions of compounds of formula IV or compounds of formula XI with compounds of formula X are known (see R.R. Wittekind et. al., "Ring Cleavage Reactions of trans-2-Amino-3α,4,5,6,7,7α-hexahydrobenzoxazole", J. Org. Chem., 26, 444 (1961)).

Compounds of formula III may be prepared by reacting compounds of formula

XV

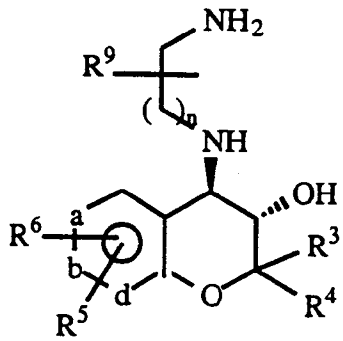

with a thiocarbonylation agent such as 1, 1'-thiocarbonyldiimidazole to form compounds of formula

XVI

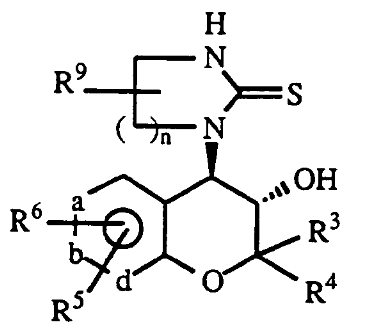

Subsequent treatment of the compounds of formula XVI with a carbodiimide such as WSC, provides compounds of formula III.

The preferred compounds of formula I are those wherein
a, b, d and all carbon atoms;
$R^1$ is hydrogen;
$R^2$ is aryl;
$R^3$ is alkyl;
$R^4$ is alkyl;
$R^5$ is hydrogen; and
$R^6$ is -CN;

The preferred compounds of formula II are those wherein
a, b, d and all carbon atoms;
$R^3$ is alkyl;
$R^4$ is alkyl;
$R^5$ is hydrogen; and
$R^6$ is -CN;
$R^7$ is hydrogen; and
$R^8$ is aryl.

The preferred compounds of formula III are those wherein
a, b, d and all carbon atoms;
$R^3$ is alkyl;
$R^4$ is alkyl;
$R^5$ is hydrogen;
$R^6$ is -CN; and
$R^9$ is hydrogen.

In preparing compounds of formulae I, II or III as described above, it may be necessary to protect any amine, hydroxy or thiol groups during the reaction with protecting groups as known in the art.

The starting materials of formula IV, XI and XV and methods of preparing them are disclosed in U.S. Patent

No. 5,140,031, incorporated herein by reference.

Preferred compounds of formulae V and XII include substituted alkyl and aryl isocyanide dihalides such as substituted phenyl isocyanide dichlorides. Most preferred compounds of formula V and XII include 4-chlorophenyl isocyanide dichloride. Substituted alkyl and aryl isocyanide dihalides are known (E. Kühle, "Carbonic Acid Derivatives from Formamides", Angew, Chem. Int, Ed., (1962), 1, 647-652; D. Ferchland et al., "Process for the Preparation of Aryl Isocyanide-Dichlorides", U.S. Patent No. 4,401,603; and E. Kühle et al., "New Methods of Preparative Organic Chemistry-Reactions of Isocyanide Dihalides and their Derivatives", Angew. Chem. Int. Ed., (1969), 8, 20-34).

Exemplary potassium channel activators preparable employing the compounds of formula I, II or III include those described in U.S. Patent No. 5,140,031, namely, the pyranyl cyanoguanidine derivatives of the formula

XVII

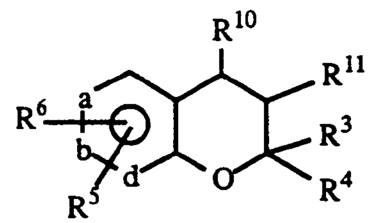

were a, b, d, $R^3$ $R^4$, $R^5$ and $R^6$ are as defined for formula I and

$R^{10}$ is

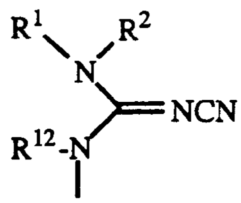

or

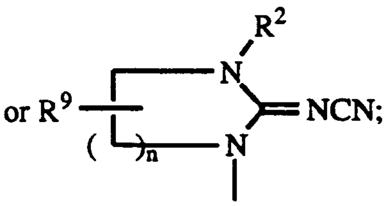

;

where

$R^9$ is as previously defined.

$R^{11}$ is hydrogen, hydroxy, $-OCOCH_3$;

$R^{12}$ is hydrogen, alkyl, alkenyl, aryl, arylalkyl, cycloalkyl or cycloalkylalkyl.

Preferred compounds of formula XVII are those where $R^{10}$ is

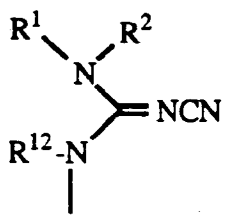

,

and $R^1$ is mono- or di- substituted phenyl.

An exemplary method of preparing the compounds of formula XVII where $R^{11}$ is hydroxy, using the intermediates of formulae I, II, or III prepared as disclosed herein includes treatment with cyanamide in a solvent such as an alcohol, for example, hot isopropyl alcohol or ethanol, or acetonitrile, optionally in the presence of a base such as triethylamine or 2,6-lutidine.

Compounds of formula XVII where $R^{11}$ is $-OC(O)CH_3$ may be prepared by acetylation of the compounds of formula XVII where $R^{11}$ is hydroxy.

Compounds of formula XVII where $R^{11}$ is hydrogen may be prepared by dehydration of the compounds of formula XVII where $R^{11}$is hydroxy, followed by reduction by procedures known in the art.

The following examples and preparations describe the manner and process of making and using the preferred embodiments of the invention and are illustrative rather than limiting. It should be understood that there may be other embodiments which fall within the spirit and scope of the invention as defined by the claims appended hereto.

Example 1

(3aS-trans)-2-[(4-Chlorophenyl)amino]-3a,4,9b-trihydro-4,4-dimethy-2H-[1]benzopyrano[4,3-d]-oxazole-8-carbonitrile

A. (3S-trans)-4-Amino-3,4-dihydro-3-hydroxy-2,2-dimethyl-2H-pyran-6-carbonitrile

A suspension of (1aS-cis)-1a,7b-dihydro-2,2-dimethyl-2H-oxireno[c][1]-benzopyran-6-carbonitrile (8.0 g, 39.7 mmol, E.N. Jacobsen, et al., J. Am. Chem. Soc., 113, 7063-7064 (1991)) in 95% ethanol (80 mL) and conc. ammonium hydroxide (80 mL) was heated at 50°C while sparging with ammonia gas. After five hours, TLC analysis indicated the reaction was complete. The reaction mixture was concentrated *in vacuo* and the residue was partitioned between ethyl acetate (160 mL) and water (75 mL). The aqueous fraction was extracted with additional ethyl acetate (4 x 75 mL). The organic fractions were combined, washed with brine, and dried (magnesium sulfate). The solvent was removed *in vacuo* to give the title compounds as a colorless foam (8.67 g, 100%).

B. (3S-trans)-N-(4-Chlorophenyl)-N'-(6-cyano-3,4-dihydro-3-hydroxy-2,2-dimethyl-2H-1-benzopyran-4-yl)thiourea

To a solution of the title A compound (2.99 g) in acetonitrile (10 mL) at room temperature under argon, was added 4-chlorophenylisothiocyanate (2.5 g) rinsed in with acetonitrile (5 mL). The clear yellow solution was stirred at room temperature. After ~45 minutes a solid began to precipitate from the reaction solution which was stirred a total of three hours. The mixture was filtered to collect the solid. The solid was rinsed with 2:1 hexane/ether and dried *in vacuo* to give 2.145 g. The filtrate was concentrated *in vacuo* and the residue was crystallized from 10 mL each of methylene chloride and ether to give 2.33 g of solid. The two crops of solid were combined to give the title compound as a white solid, 4.426 g, 84.15%; m.p. 181.6°C to 181.9°C.
$^{13}C$ NMR (DMSO-$d_6$): δ 182.0, 156.2, 137.8, 132.6, 128.6, 128.3, 125.2, 124.9, 119.1, 117.9, 102.4, 80.4, 70.8, 54.0, 26.6, 18.8 ppm.

| Elemental Analysis for $C_{19}H_{18}N_3O_2ClS$ | | | | | |
|---|---|---|---|---|---|
| Calc'd: | C 58.83; | H 4.68; | N 10.83; | Cl 9.14; | S 8.27; |
| Found: | C 58.76; | H 4,64; | N 10.63; | Cl 9.24; | S 8.42. |

$[a]_D$ = -57.3° (c=0.5%, methanol)

Alternate Procedure for preparing: (3S-trans)-N-(4-Chlorophenyl)-N'-(6-cyano-3,4-dihydro-3-hydroxy-2,2-dimethyl-2H-1-benzopyran-4-yl)thiourea

To a solution of the title A compound (3S-trans)-4-Amino-3,4-dihydro-3-hydroxy-2,2-dimethyl-2H-pyran-6-carbonitrile (8.67 g, 39.8 mmol) in acetonitrile (89 mL) at 62°C was added methanesulfonic acid (2.71 mL, 41.8 mmol). The resulting slurry was stirred at 62°C for one hour and then cooled to room temperature. The mixture was filtered and the solid was washed with acetonitrile (2 x 20 mL) and hexane (2 x 200 mL) and dried *in vacuo* to give (3S-trans)-4-Amino-3,4-dihydro-3-hydroxy-2,2-dimethyl-2H-pyran-6-carbonitrile methane sulfonate salt as a colorless solid (11.25 g, 90%); m.p. 226°C to 227°C.
$[a]_D$ = +50.2° (c=1, $CH_3OH$)

A suspension of (3S-trans)-4-Amino-3,4-dihydro-3-hydroxy-2,2-dimethyl-2H-pyran-6-carbonitrile methane sulfonate salt compound (20.0 g) in acetonitrile (50 mL) under argon in a 250 mL flask fitted with a magnetic stirrer was prepared. Triethylamine (9.15 g) was added to give a clear colorless solution. 4-chlorophenylisothiocyanate (11.65 g) was added portionwise at a rate to keep the reaction temperature below 31°C. (Approximately 20 minutes required for the addition.) The mixture was stirred a total of four hours at room temperature and monitored by tlc. After dilution with 250 ethyl acetate, the mixture was washed with 0.5N hydrochloric acid (100 mL), 1:1 water-saturated sodium chloride solution (2 x 50 mL), saturated sodium bicarbonate

solution (50 mL) and then twice with saturated sodium chloride solution. The organic solution was dried over magnesium sulfate, filtered and concentrated *in vacuo* to give the crude product as a white foam (28.7 g). The product (28.7 g) was dissolved in hot acetonitrile (50 mL) and let stand at room temperature overnight to crystallize. The solid was collected, washed with 2:1 hexane-ethyl acetate (2 x 10 mL), and dried to give 11.8 g, 47.8%; m.p. 180.8°C to 181.3°C. The filtrate was concentrated and the residue crystallized from 1:1 ethyl acetate-hexane (80 mL) to give 10.1 g, 40.8%; m.p. 180.8°C to 181.3°C. A third crop gave 1.54 g, 6.24%; m.p. 180.7°C to 181.3°C. Total yield of crystalline product: 23.4 g, 94.8%.

C. (3aS-trans)-2-[(4-Chlorophenyl)amino]-3a,4,9b-trihydro-4,4-dimethy-2H-[1]benzopyrano[4,3-d]-oxazole-8-carbonitrile

A solution of the title B compound (4.0 g) in ethyl acetate (70 mL) was prepared under argon in a 200 mL flask fitted with a magnetic stirrer and argon inlet. An amount of WSC (3.26 g) was added and the mixture was stirred in a 70°C bath for two hours and the reaction was monitored by tlc. The ethyl acetate solution was decanted from the white tar-like precipitate and the flask washed with ethyl acetate (50 mL). The organic extract was washed with 0.5N hydrochloric acid (100 mL), 1:1 water-saturated sodium chloride (50 mL), sodium bicarbonate (50 mL) and brine. The organic solution was dried over magnesium sulfate, filtered and concentrated *in vacuo* to give a glass-foam product (3.45 g, 94.7%). An amount of the product (3.40 g) was dissolved in hot ethyl acetate (12 mL), diluted with hexane (24 mL) and let stand to crystallize. The product was collected, rinsed with 2:1 hexane/ethyl acetate and dried to give the title compound as fine white needles (2.38 g, 65.3%); m.p. 175.1°C to 175.5°C.
The $^{1}H$ NMR and $^{13}C$ NMR in DMSO-d at 110°C or in 14% $CD_3COOD$ in $CDCl_3$ were consistent with the desired product. The endocyclic position of the double bond was confirmed by X-ray analysis.

| Elemental Analysis for $C_{19}H_{16}N_3O_2Cl$ | | | | |
|---|---|---|---|---|
| Calc'd: | C 64.50; | H 4.56; | N 11.88; | Cl 10.02; |
| Found: | C 64.55; | H 4.46; | N 11.74; | Cl 9.97. |

Example 2

(3aS-trans)-2-[(4-Chlorophenyl)amino]-3a,4,9b-trihydro-4,4-dimethy-2H-[1]benzopyrano[4,3-d]-oxazole-8-carbonitrile

To a solution of the compound (3S-trans)-4-Amino-3,4-dihydro-3-hydroxy-2,2-dimethy-2H-pyran-6-carbonitrile methane sulfonate salt, prepared in Example 1, (1.0g, 3.18 mmol) in absolute ethanol (5 mL) at room temperature under argon, was added diisopropylethylamine (2.40 mL, 13.78 mmol, 4.33 eq). To the resulting solution was added 4-chlorophenyl isocyanide dichloride (1.0g, 4.80 mmol, 1.50 eq). After heating at 43°C for 24 hours, the resulting mixture was diluted with toluene (~80 mL) and washed with water, 5% aqueous $NaHSO_4$ (25 mL), 1N $NaHCO_3$, and brine. After drying (magnesium sulfate), the solvent was removed *in vacuo* to give a light yellow solid. This solid was then slurried with heptane (10 mL). The solid was collected by filtration, washed with heptane, and dried to give the crude product (1.14 g, HPLC HI=94.0%). This crude product (1.097 g) was recrystallized from ethyl acetate (3 mL) and heptane (5 mL) to give the product as a colorless solid: 754 mg (69%); HPLC HI=99.5%; $[a]_D$=-106.9° (c=0.55, $CH_3OH$).

Example 3

(3S-trans)-N-(4-Chlorophenyl)-N"-cyano-N'-(6-cyano-3,4-dihydro-3-hydroxy-2,2-dimethyl-2H-1-benzopyran-4-yl)guanidine

A mixture of the title compound of Example 1, (3aS-trans)-2-[(4-chlorophenyl)amino]-3a,4,9b-trihydro-4,4-dimethy-2H-[1]benzopyrano[4,3-d]-oxazole-8-carbonitrile (4.00 g) and cyanamide (1.42 g) in isopropanol (22 mL) was prepared under argon in a 100 mL flask equipped with a magnetic stirrer and a condenser. An amount of 2,6-lutidine (1.28 g) was added and the mixture heated in a 95°C bath to give a clear colorless solution. The solution was heated for 16 hours in the 95°C bath and monitored by HPLC. The solution was diluted with ethyl acetate (150 mL) and washed with water (50 mL) containing 1N hydrochloric acid (14 mL). The ethyl acetate solution was diluted with ethyl acetate (25 mL) and washed with 5:2 water-brine (70 mL), and then saturated sodium bicarbonate and brine. The organic layer was dried over magnesium sulfate, filtered, and concentrated

*in vacuo* to give an off-white amorphous solid, 4.91 g with HPLC HI=97.8%. This material was dissolved in hot 95% ethanol (60 mL) diluted with hot water (62 mL) and stirred in a 63°C bath for four hours as a white solid precipitated. The mixture was let stir at room temperature overnight. The mixture was filtered and the solid was washed with water (3 x 20 mL) and dried (eight hours in the air, 16 hours under nitrogen) to give 4.07 g (90.6%, corrected for 0.1 meq water), HPLC HI=99.2%, $[a]_D$=-33.3° (c=0.5, methanol). Recrystallization:
A sample of the solid (1.4 g) was dissolved in hot 95% ethanol (15 mL), diluted with hot water (15.5 mL) and stirred in a 63°C bath in an open flask for three hours. The mixture was stirred at room temperature overnight. The mixture was filtered, and the isolated solid was washed with 30% ethanol (10 mL) in water and water (3 x 10 mL). The solid was dried under nitrogen atmosphere overnight at room temperature to give the title compound as a white solid, 1.33 g. HPLC HI=99.76%.

| Elemental Analysis for $C_{20}H_{18}N_5O_2Cl$ (395.85) | | | | | |
|---|---|---|---|---|---|
| Calc'd: | C 60.68; | H 4.58; | N 17.69; | Cl 8.96; | KF; |
| Found: | C 60.65; | H 4,64; | N 17.59; | Cl 8.90; | KF 0.17. |

## Claims

**1.** Compounds of the formula

I

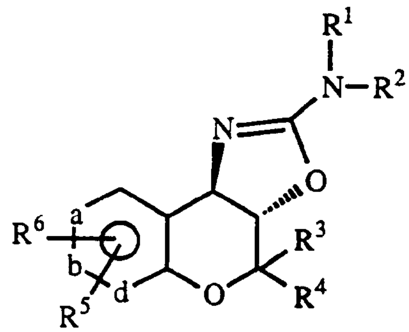

;

II

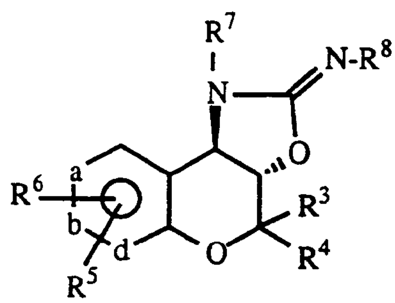

;

or

III

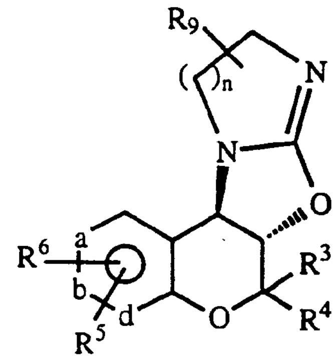

wherein a, b, and d are all carbon atoms or one of a, b and d is a nitrogen atom or -N(O)- and the others are carbon atoms;

$R^1$ and $R^2$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or $R^1$ and $R^2$ taken together with the nitrogen atom to which they are attached form 1-pyrrolidinyl, 1-piperidinyl, 1-azepinyl, 4-morpholinyl, 4-thiamorphilinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

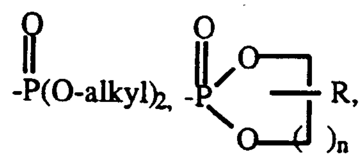

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^7$ and $R^8$ are each independently selected from hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^9$ is hydrogen, alkyl, alkenyl, aryl, arylalkyl, cycloalkyl, (cycloalkyl)alkyl or an aryl group fused to 2 adjacent carbon atoms; and

n is an integer of 1 to 3.

**2.** The compounds of Claim 1 wherein in formula I

a, b, d and all carbon atoms;
$R^1$ is hydrogen;
$R^2$ is aryl;
$R^3$ is alkyl;
$R^4$ is alkyl;
$R^5$ is hydrogen; and
$R^6$ is -CN;

in formula II

a, b, d and all carbon atoms;
$R^3$ is alkyl;
$R^4$ is alkyl;
$R^5$ is hydrogen;

$R^6$ is -CN;
$R^7$ is hydrogen; and
$R^8$ is aryl; and

in formula III

a, b, d and all carbon atoms;
$R^3$ is alkyl;
$R^4$ is alkyl;
$R^5$ is hydrogen;
$R^6$ is -CN; and
$R^9$ is hydrogen.

3. A compound of Claim 1 which is (3aS-trans)-2-[(4-Chlorophenyl)-amino]-3a,4,9b-trihydro-4,4-dimethy-2H-[1]benzopyrano[4,3-d]-oxazole-8-carbonitrile or a salt thereof.

4. A process for the preparation of compounds of the formula

I

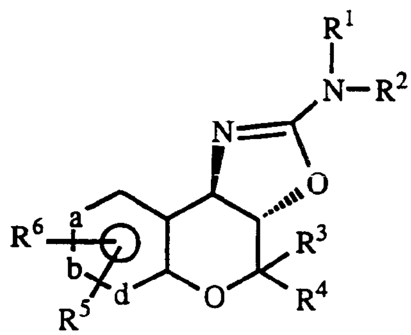

wherein a, b, and d are all carbon atoms or one of a, b and d is a nitrogen atom or -N(O)- and the others are carbon atoms;

$R^1$ is hydrogen and $R^2$ is alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

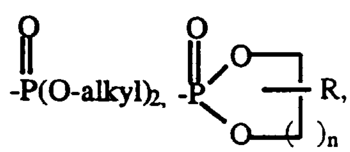

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$; and

n is an integer of 1 to 3; comprising the step of reacting a compound of formula

IV

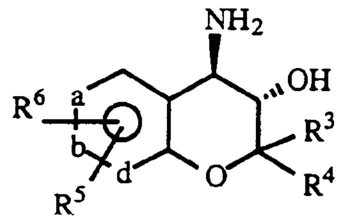

with an isocyanide dihalide of formula

V $R^2\text{-}N{=}C(X)_2$

where X is a halogen in a solvent containing a tertiary amine.

5. A process for the preparation of compounds of the formula

I

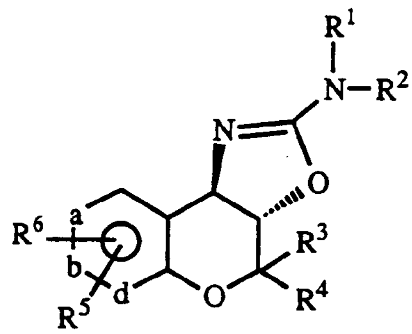

wherein a, b, and d are all carbon atoms or one of a, b and d is a nitrogen atom or -N(O)- and the others are carbon atoms;

$R^1$ is hydrogen and $R^2$ is alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl,$-SO_2$alkyl,

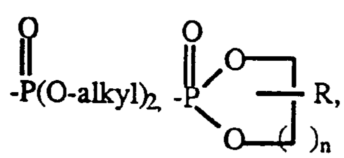

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$; and

n is an integer of 1 to 3; comprising the step of reacting a compound of formula

IV

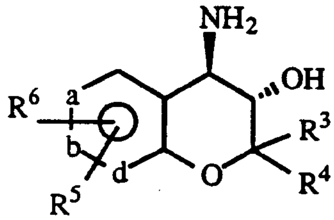

with an isothiocyanate of the formula

VI $R^2$-N=C=S

to provide a thiourea of formula

VII

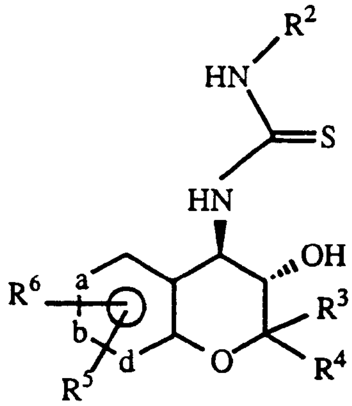

and subsequently treating said thiourea of formula VII with a carbodiimide.

6. A process for the preparation of compounds of the formula

I

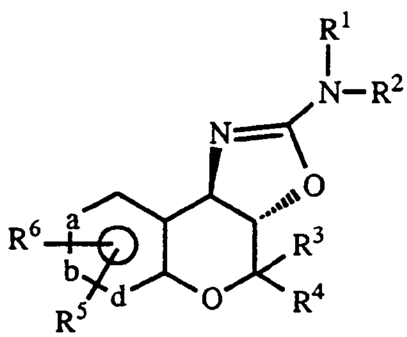

wherein a, b, and d are all carbon atoms or one of a, b and d is a nitrogen atom or -N(O)- and the others are carbon atoms;

$R^1$ and $R^2$ are each independently selected from alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino, or $R^1$ and $R^2$ taken together with the nitrogen atom to which they are attached form 1-pyrrolidinyl, 1-piperidinyl, 1-azepinyl, 4-morpholinyl, 4-thiamorphilinyl, 1-piperazinyl, 4-alkyl-1-piperazinyl or 4-arylalkyl-1-piperazinyl, wherein each of the so-formed groups can be substituted with alkyl, alkoxy, alkylthio, halogen or trifluoromethyl;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl,

-CN, $-NO_2$, -COR, -COOR, -CONHR, -CONRR', $-CF_3$, S-alkyl, -SOalkyl, $-SO_2$alkyl,

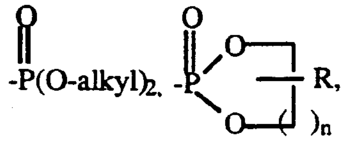

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$; and

n is an integer of 1 to 3; comprising the step of reacting a compound of formula

IV

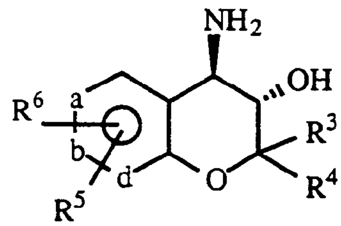

with a compound of formula

VIII

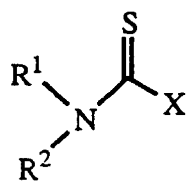

where X is a halogen, to form a compound of formula

IX

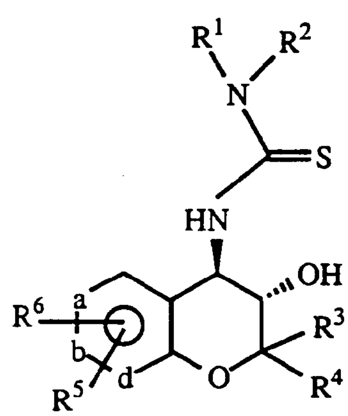

and subsequently treating said thiourea of formula IX with a carbodiimide.

**7.** A process for the preparation of compounds of the formula

I

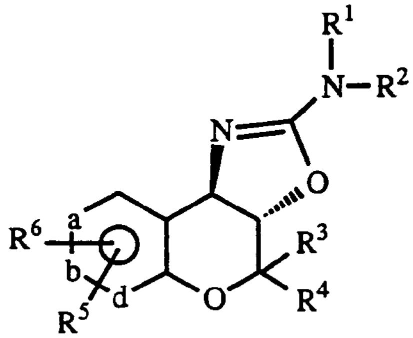

wherein a, b, and d are all carbon atoms or one of a, b and d is a nitrogen atom or -N(O)- and the others are carbon atoms;

$R^1$ and $R^2$ are each hydrogen;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

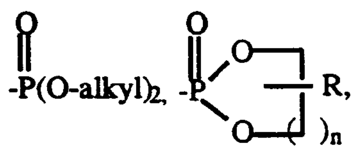

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$; and

n is an integer of 1 to 3; comprising the step of reacting a compound of formula

IV

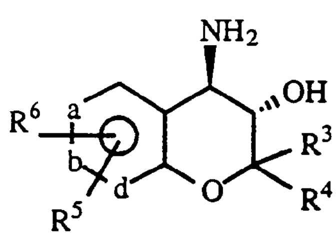

with a compound of formula

X X-CN

where X is a halogen.

8. A process for the preparation of compounds of the formula

II

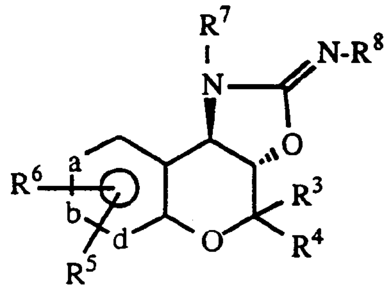

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

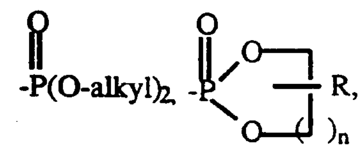

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^7$ is hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^8$ is alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino; and

n is an integer of 1 to 3; comprising the step of treating a compound of formula

XI

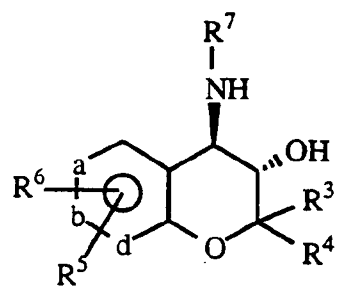

with a isocyanide dihalide of formula

XII $R^8\text{-N=C(X)}_2$

where X is a halogen in a solvent containing a tertiary amine.

9. A process for the preparation of compounds of the formula

II

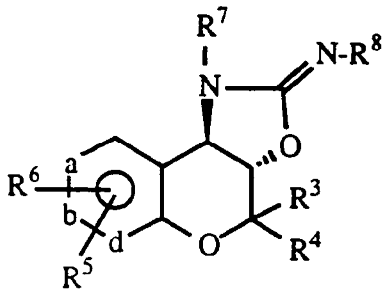

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

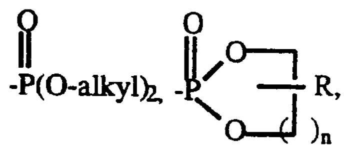

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^7$ is hydrogen, alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino;

$R^8$ is alkyl, alkenyl, aryl, (heterocyclo)alkyl, heterocyclo, arylalkyl, cycloalkyl and (cycloalkyl)alkyl, substituted alkyl wherein the substituents include alkoxy, alkylthio and substituted amino; and

n is an integer of 1 to 3; comprising the step of treating a compound of formula

XI

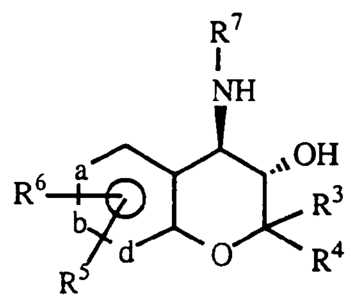

with an isothiocyanate of formula

XIII $R^8-N=C=S$

to provide a thiourea of formula

XIV

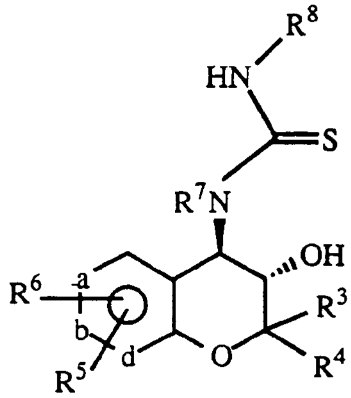

and subsequently treating said thiourea of formula XIV with a carbodiimide.

**10.** A process for the preparation of compounds of the formula

III

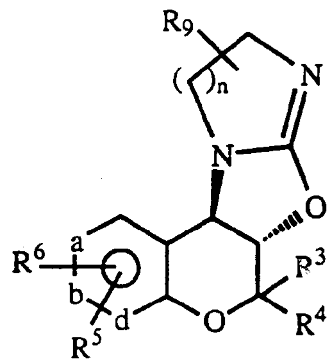

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

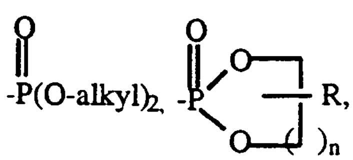

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^9$ is hydrogen, alkyl, alkenyl, aryl, arylalkyl, cycloalkyl, (cycloalkyl)alkyl or an aryl group fused to 2 adjacent carbon atoms; and

n is an integer of 1 to 3; comprising the step of reacting a compound of formula

XV

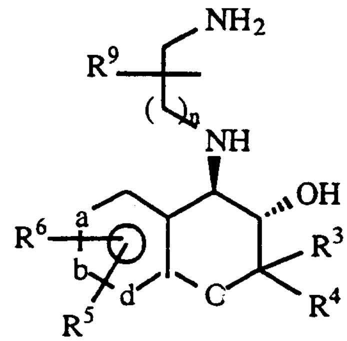

with a thiocarbonylation agent to form compounds of formula

XVI

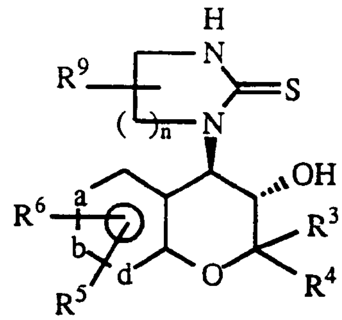

which is then treated with a carbodiimide.

**11.** A process for the preparation of compounds of formula

XVIIa

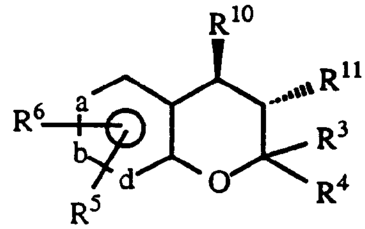

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

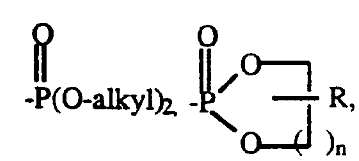

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl,

haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

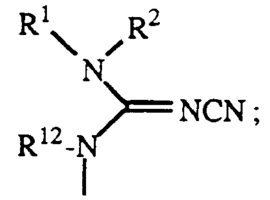

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula I

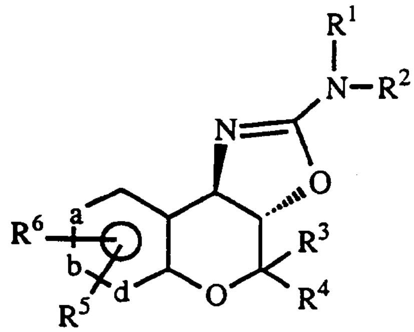

;

and

(B) converting said compound of the formula I prepared in step (A) to said compound of formula XVIIa, with the proviso that said compound of the formula I is as defined in, and is prepared by, the process of Claim 4.

**12.** A process for the preparation of compounds of formula

XVIIa

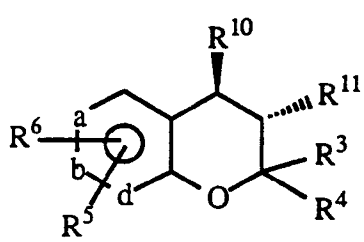

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

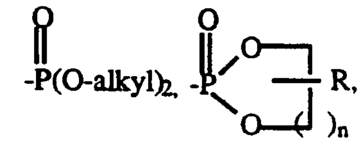

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

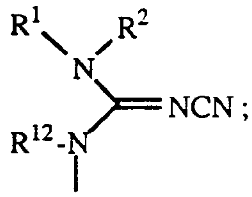

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula I

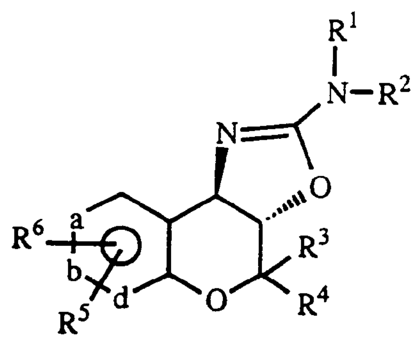

;

and

(B) converting said compound of the formula I prepared in step (A) to said compound of formula XVIIa, with the proviso that said compound of the formula I is as defined in, and is prepared by, the process of Claim 5.

**13.** A process for the preparation of compounds of formula

**XVIIa**

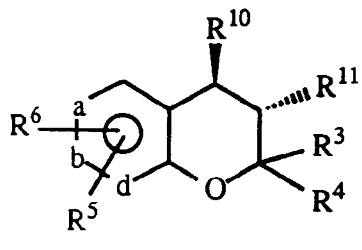

wherein a, b, and d are all carbon atoms or one of a, b and d is a nitrogen atom or -N(O)- and the others

are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

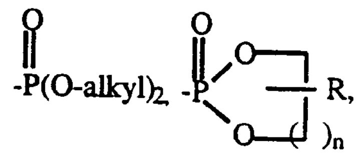

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

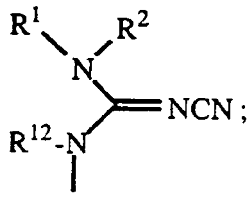

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula I

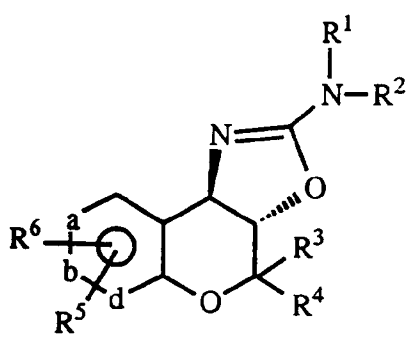

;

and

(B) converting said compound of the formula I prepared in step (A) to said compound of formula XVIIa, with the proviso that said compound of the formula I is as defined in, and is prepared by, the process of Claim 6.

**14.** A process for the preparation of compounds of formula

XVIIa

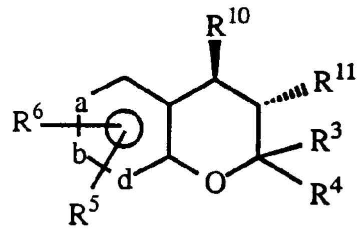

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', - $CF_3$, S-alkyl, -SOalkyl, $-SO_2$alkyl,

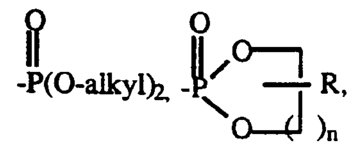

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

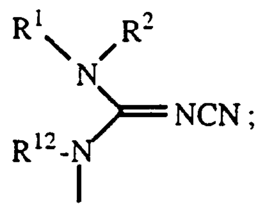

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula I

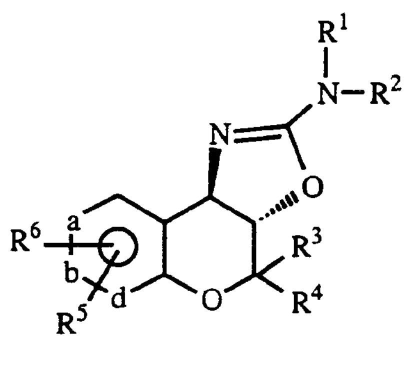

;

and

(B) converting said compound of the formula I prepared in step (A) to said compound of formula XVIIa,

with the proviso that said compound of the formula I is as defined in, and is prepared by, the process of Claim 7.

**15.** The process as recited in Claim 11 wherein a compound of the formula

XVIIa

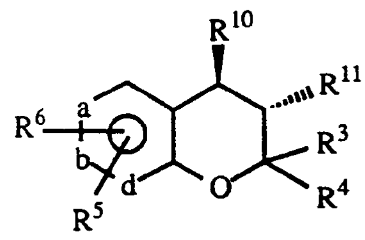

where $R^{10}$ is

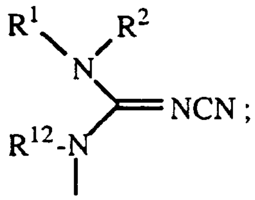

and $R^1$ is mono- or di- substituted phenyl is prepared.

**16.** The process as recited in Claim 15 wherein the compound of formula XVII is (3S-trans)-N-(4-Chlorophenyl)-N''-cyano-N'-(6-cyano-3,4-dihydro-3-hydroxy-2,2-dimethyl-2H-1-benzopyran-4-yl)guanidine or a pharmaceutically acceptable salt thereof.

**17.** A process for the preparation of compounds of formula

XVIIa

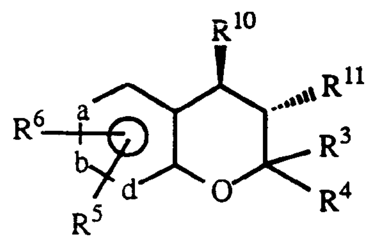

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

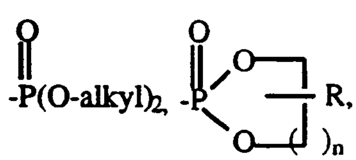

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl,

haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

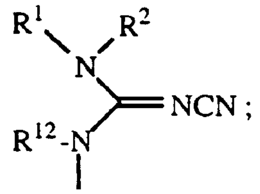

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula

II

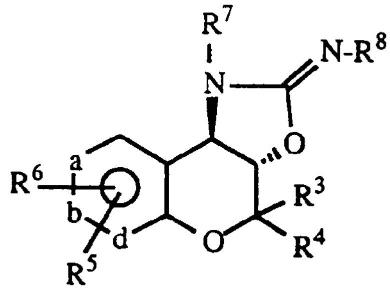

;

and

(B) converting said compound of the formula II prepared in step (A) to said compound of formula XVIIa, with the proviso that said compound of the formula II is as defined in, and is prepared by, the process of Claim 8.

**18.** The process as recited in Claim 17 wherein the compound of formula XVII is (3S-trans)-N-(4-Chlorophenyl)-N"-cyano-N'-(6-cyano-3,4-dihydro-3-hydroxy-2,2-dimethyl-2H-1-benzopyran-4-yl)guanidine or a pharmaceutically acceptable salt thereof.

**19.** A process for the preparation of compounds of formula

**XVIIa**

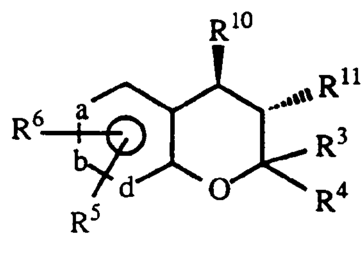

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

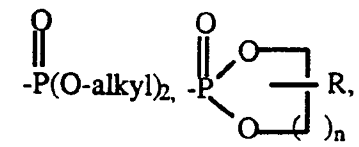

halogen, amino, substituted amino -OH, -O-alkyl, $-OCF_3$, $-OCH_2CF_3$, -OCOalkyl, -OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

$R^{10}$ is 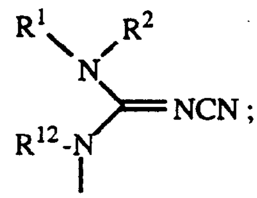

$R^{11}$ is hydrogen, hydroxy, $-OCOCH_3$;

$R^{12}$ is hydrogen, alkyl, alkenyl, aryl, arylalkyl, cycloalkyl or cycloalkylalkyl; and n is an integer of 1 to 3; comprising the steps of

(A) preparing a compound of the formula

II

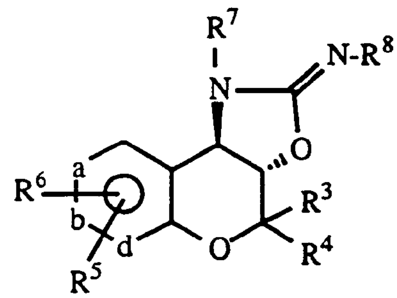

;

and

(B) converting said compound of the formula II prepared in step (A) to said compound of formula XVIIa, with the proviso that said compound of the formula II is as defined in, and is prepared by, the process of Claim 9.

**20.** The process as recited in Claim 19 wherein the compound of formula XVII is (3S-trans)-N-(4-Chlorophenyl)-N"-cyano-N'-(6-cyano-3,4-dihydro-3-hydroxy-2,2-dimethyl-2H-1-benzopyran-4-yl)guanidine or a pharmaceutically acceptable salt thereof.

**21.** A process for the preparation of compounds of formula

XVIIa

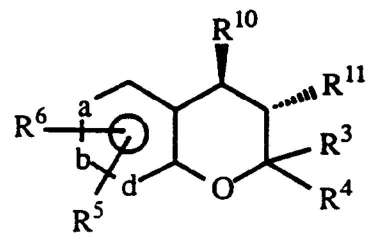

wherein a, b, and d are all carbon atoms or one of a, b and d is a nitrogen atom or -N(O)- and the others are carbon atoms;

$R^3$ and $R^4$ are each independently hydrogen, alkyl or arylalkyl, or, $R^3$ and $R^4$ taken together with the carbon atom to which they are attached form a 5- to 7-membered carbocyclic ring;

$R^5$ is selected from hydrogen, alkyl, haloalkyl, alkenyl, alkynyl, cycloalkyl, arylalkyl, cycloalkylalkyl, -CN, $-NO_2$, -COR, -COOR, -CONHR, -CONRR', $-CF_3$, S-alkyl, -SOalkyl, $-SO_2$alkyl,

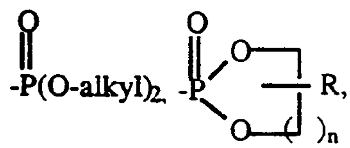

halogen, amino, substituted amino -OH, -O-alkyl, $-OCF_3$, $-OCH_2CF_3$, -OCOalkyl, OCONRalkyl, -NRCOalkyl, -NRCOOalkyl and -NRCONRR' wherein R and R' in each of the above groups can be hydrogen, alkyl, haloalkyl, aryl, arylalkyl, cycloalkyl, or (cycloalkyl)alkyl;

$R^6$ is selected from hydrogen, alkyl, -OH, -O-alkyl, amino, substituted amino, -NHCOR (wherein R is as defined above), -CN, and $-NO_2$;

$R^{10}$ is

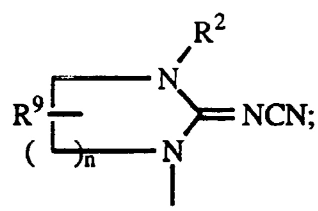

where $R^9$ is hydrogen, alkyl, alkenyl, aryl, arylalkyl, cycloalkyl, cycloalkylalkyl or an aryl group fused to 2 adjacent carbon atoms;

$R^{11}$ is hydrogen, hydroxy, $-OCOCH_3$; and

n is an integer of 1 to 3; comprising the steps of

(A) preparing a compound of the formula

III

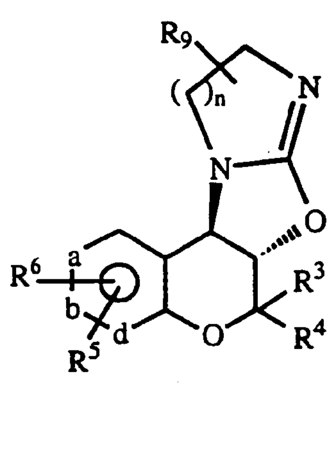

;

and
(B) converting said compound of the formula III prepared in step (A) to said compound of formula XVIIa, with the proviso that said compound of the formula III is as defined in, and is prepared by, the process of Claim 10.